# EUROPEAN PATENT SPECIFICATION

(11) **EP 1 169 044 B1**
(45) Date of publication and mention of the grant of the patent: **10.09.2003**
(21) Application number: 00921217.6
(22) Date of filing: 16.03.2000
(51) Int. Cl.: A61K 31/702, A61K 31/715, A61P 1/04

(54) **USE OF FUCOSYLATED SIALYLATED N-ACETYL LACTOSAMINE CARBOHYDRATE STRUCTURES FOR INHIBITION OF BACTERIAL ADHERENCE**
VERWENDUNG VON FUCOSILIERTEN SIALILIERTEN N-ACETYL-LACTOSAMIN-KOHLENHYDRATSTRUKTUREN ZUR HEMMUNG DER BAKTERIELLEN ADHÄSION
STRUCTURES GLUCIDIQUES DE N-ACETYL LACTOSAMINE FUCOSYLE SIALYLE PERMETTANT D'INHIBER L'ADHESION BACTERIENNE

(30) Priority: 19.03.1999 SE 9901007
(43) Date of publication of application: 09.01.2002
(73) Proprietor: Borén, Thomas, 906 43 Umea (SE)
(72) Inventor: BOREN, Thomas, 906 43 Umea (SE); HAMMARSTRÖM, Lennart, S-141 44 Huddinge (SE); KARLSSON, Karl-Anders, S-411 43 Göteborg (SE); TENEBERG, Susann, S-430 63 Hindas (SE)
(74) Representative: Larfeldt, Helene
(86) International application number: SE0000514
(87) International publication number: WO00056343

(56) References cited:
- WO-A1-95/00527
- WO-A1-95/23605
- HIROYOSHI OTA ET AL.: 'Helicobacter pylori infection produces reversible glycosylation changes to gastric mucins' VIRCHOWS ARCH., vol. 433, 1998, pages 419 - 426, XP002928801
- THOMAS BOREN ET AL.: 'Attachment of helicobacter pylori to Human Gastric Epithelium Mediated by Blood Group Antigens' SCIENCE, vol. 262, 17 December 1993, pages 1892 - 1895, XP002928802

## Description

The present invention relates to the use of at least one fucosylated sialylated N-acetyl lactosamine carbohydrate structure, such as sialyl-Lewis antigen carbohydrate structure for the preparation of pharmaceutical compositions for the treatment or prophylaxis in humans of conditions involving infection by *Helicobacter pylori* or related organisms in the human gastrointestinal mucosa, as well as a medicament for treating such conditions using fucosylated sialylated lactosamine structures, such as said sialyl-Lewis antigen carbohydrate structures.

### Background of the invention

*H. pylori*, the human specific gastric pathogen, has emerged as the causative agent in chronic active gastritis and peptic ulcer disease. The chronic infection has been correlated to the development of gastric cancer, one of the most common forms of cancer in humans (reviewed in M. J. Blaser. *Sci. Amer.* **2**, 92, 1996). *H. pylori* colonizes the human gastric mucosa by adherence to the mucous epithelial cells and the mucus layer lining the gastric epithelium. These adherence properties protect the bacteria from the extreme acidity of the gastric lumen and displacement from the stomach by forces such as gastric emptying. Central to current thinking in pathogenesis is the importance of adherence for colonization or disease. Bacteria express adhesion molecules that recognize specific carbohydrates on the epithelial cell surface and mucin molecules in the mucus lining.

It has previously been demonstrated that the fucosylated blood group antigens Lewis b and H-1 to mediate adherence of *H. pylori* to human gastric epithelial cells *in situ* (T. Borén et al., *Science 1993,* 262, 1892). The fucosylated blood group antigens H-1 and Lewis b are typically found on erythrocytes where they define the O phenotype in the ABO blood group system, but they are also expressed on the epithelial cell surfaces. A relationship between histo-blood group antigens and the development of duodenal ulcer has received attention since the discovery of Aird *et al*. 1954. *Br Med J*, 315, that the frequency of individuals with blood group 0 is strikingly high in patients suffering from peptic ulcer. The *H. pylori* specificity for the Lewis b and H-1 antigens as receptors, could possibly explain the higher prevalence for peptic ulcer disease in blood group O individuals, since the level of available *H. pylori* receptors might be reduced in individuals of blood group A and B phenotypes (T. Borén and P. Falk, *Science (*letter), 1994, 264, 1387., T. Borén and P. Falk (1994) *Scientific American, Science & Medicine,* Sept/Oct. 28-37).

WO 95/23605 discloses a method for treating and inhibiting gastric and duodenal ulcers, comprising administering a pharmaceutical composition comprising an oligosaccharide, in particular NeuAc-α(2-3)pGal-β(1-4)Glc. The compounds of the present invention are however not identified.

WO 98/55630 discloses a bacterial α1,3-fucosyltransferase gene and the deduced amino acid sequence. The resulting polypeptide is said to be useful in the production of oligosaccharides such as sialyl-Lewis x, which are structurally similar to certain tumor-associated carbohydrate antigens found in mammals.

WO 95/35303 discloses methods of synthesis useful for the preparation of cyclitol and/or carbohydrate conjugates and carbocyclic analogs thereof. Sialyl Lewis X is mentioned as a cellular surface oligosaccharide found in increased amounts in patients suffering from breast, pancreatic and gastrointestinal cancer.

The problem underlying the present invention is that of finding clinically significant and efficient substances that influence bacterial adherence and in particular the adherence of *H. pylori*, a prevalent pathogen in humans, to the human gastric mucosa. A further problem is to make available new and efficient medicaments for the treatment or prophylaxis of *H. pylori* infections.

### Summary of the invention

The present invention solves the above problems in that it makes available a novel use of fucosylated sialylated N-acetyl lactosamine glycoconjugates, such as sialyl-Lewis x structures.

### Description of the figures

The present invention will be described in closer detail below, with reference to the attached examples and figures, in which:

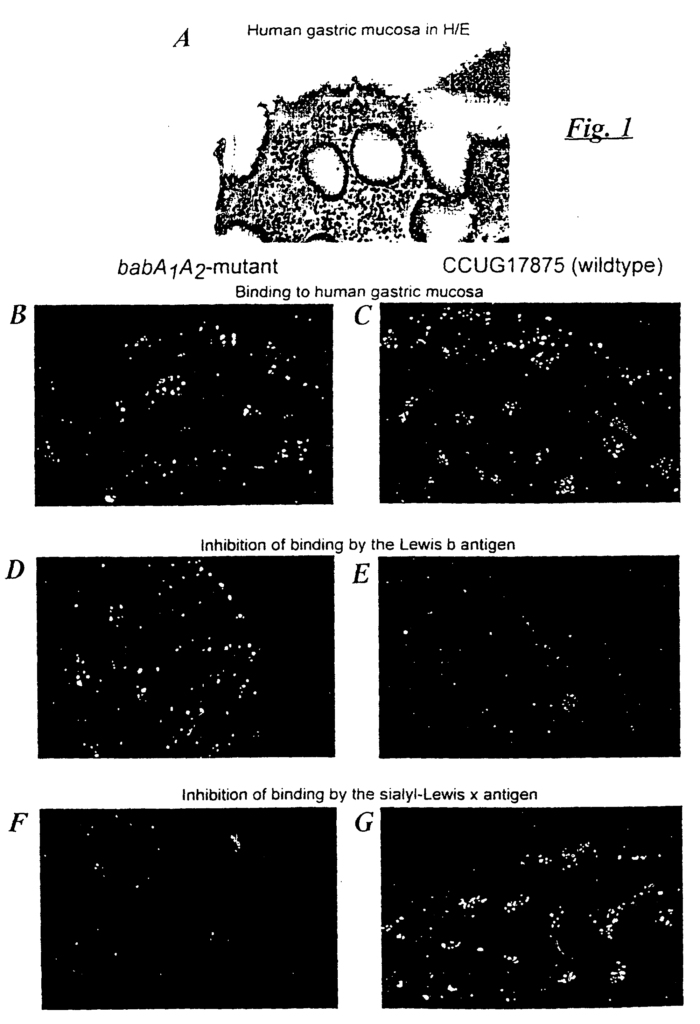

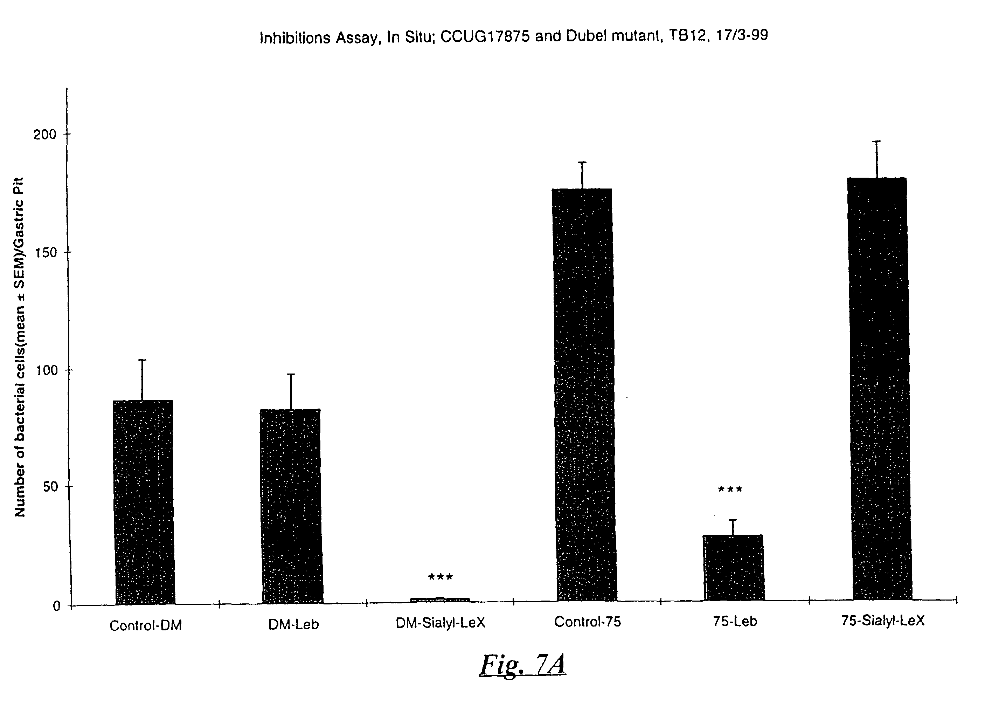

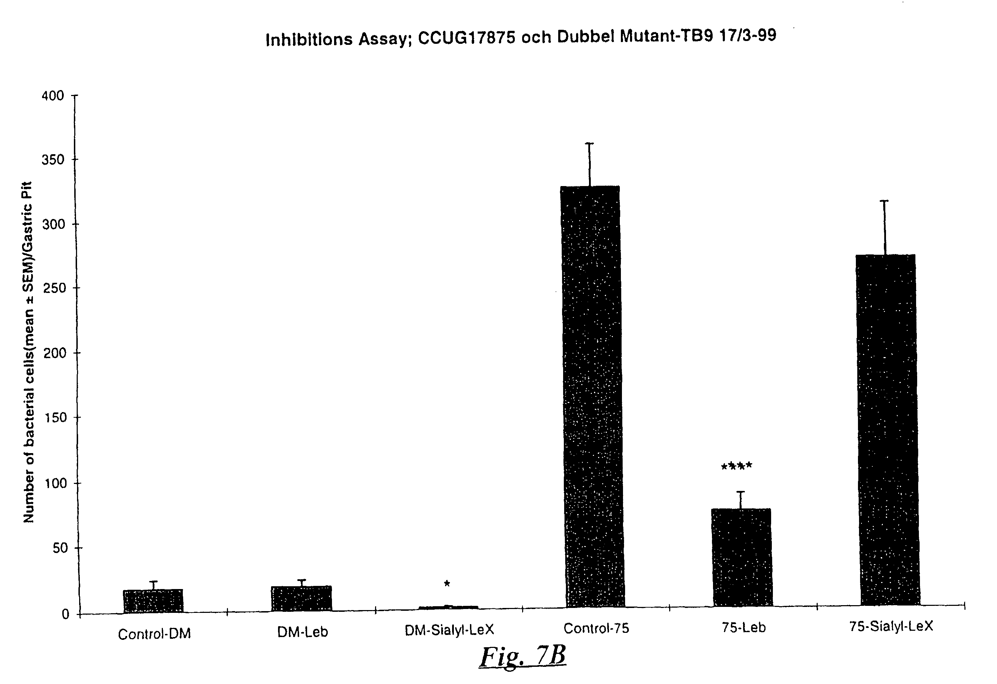

**Fig. 1** and **Fig. 7A, B** show bacterial *in situ* adhesion to human gastric mucosa and inhibition assay: *In situ* adherence analysis of the receptor specificity of *H. pylori* (CCUG 17875 and the *babA1A2-* -mutant). Bacteria were pre-incubated with the Lewis b conjugate in 10µg/ml concentration, or the sialyl-Lewis conjugate in 20µg/ml concentration. (A) Section of human gastric mucosa stained with hematoxylin/eosin. (B, C) Non-inhibited *H. pylori babA1A2-* mutant and CCUG 17875, respectively, binding to human gastric epithelium. (D, E) Inhibition experiments with Lewis b antigen-, and sialyl-Lewis x antigen-neoglycoconjugate, respectively (F, G).

For Figure 1A-1F, biopsy no.12 (Fig. 7A) was used, with a moderate degree of inflammation cell infiltration. For Fig. 7B, a biopsy no. 9 was used, with less inflammation infiltrate and almost devoid of receptors for the *babA1A2*-mutant.

Reduction in bacterial binding was estimated by counting the number of adherent bacteria in 10 different fields under 200X magnification in two independent inhibition experiments. The control where bacteria *were not* pre-incubated with glycoconjugates was defined as 100% binding.

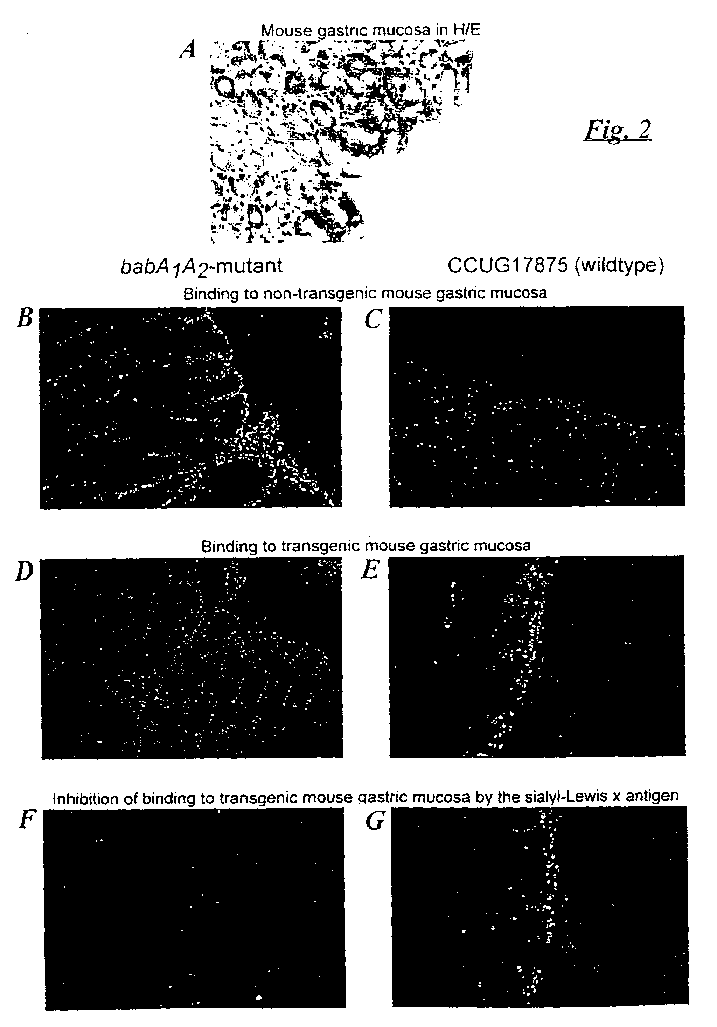

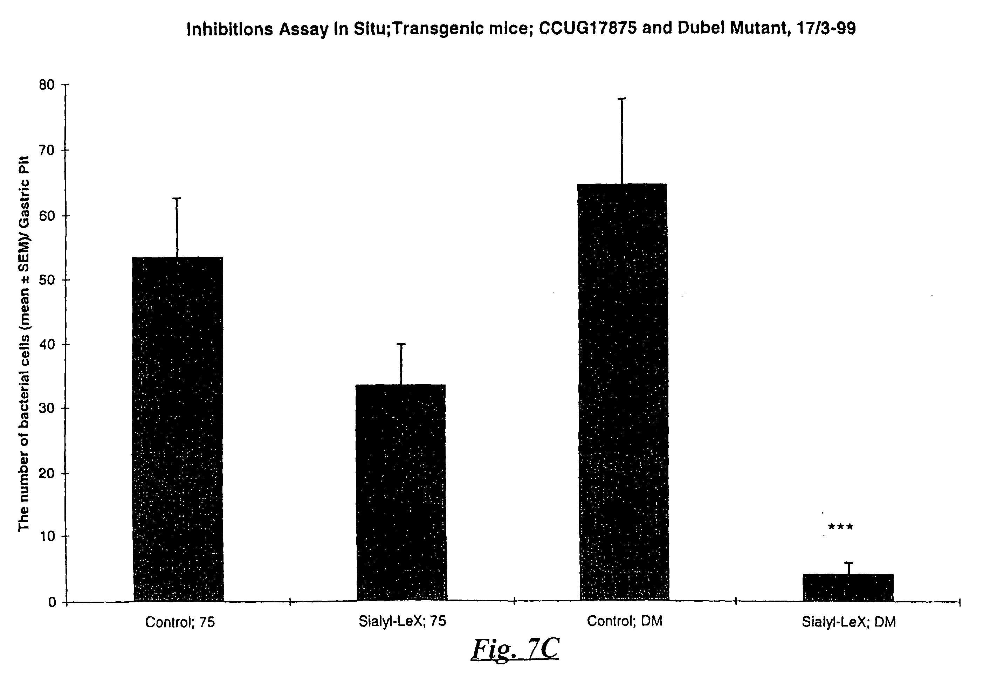

**Fig. 2** and **7C** show bacterial *in situ* adhesion to mouse gastric mucosa and inhibition assay: In situ adherence analysis of the receptor specificity of *H. pylori*. CCUG 17875 and the *babA1A2*- -mutant. (A) Section of mouse gastric mucosa stained with hematoxylin/eosin. (B, C) Non-inhibited *H. pylori babA1A2*-mutant and CCUG 17875 overlaid on non-transgenic gastric tissue (B, C). and the "Lewis b mouse" transgenic gastric tissue (D, E), respectively. Inhibition experiments by the "Lewis b mouse" transgenic gastric tissue with sialyl-Lewis x neoglycoconjugate (F, G), ion concentrations as described in Fig. 1.

For Fig. 7C the experimental system was as above, and reduction in bacterial binding was estimated by counting the number of adherent bacteria in 10 different fields under 200X magnification in two independent inhibition experiments.

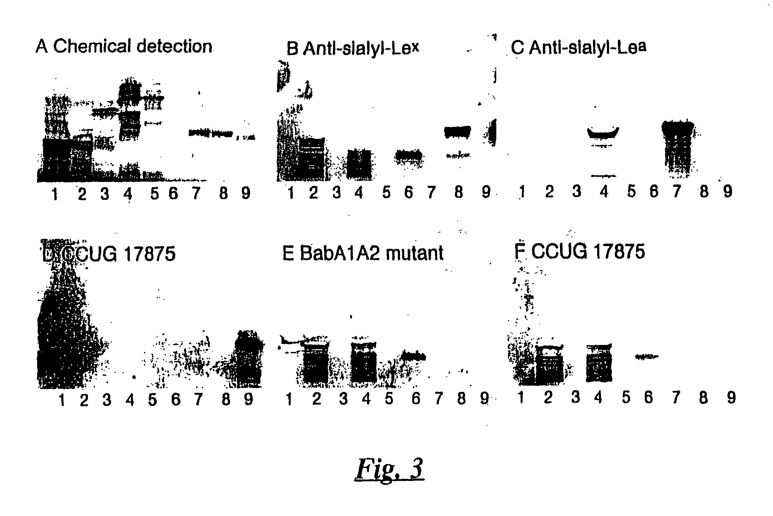

**Fig. 3** shows the results of analyses of *H. pylori* binding to HPTLC separated sialylated glycolipids: Binding of *Helicobacter pylori* and monoclonal antibodies directed against sialyl-Lewis x and sialyl-Lewis a to glycosphingolipids on thin-layer chromatograms. The glycosphingolipids were separated and (A) visualised with anisaldehyde. Duplicate chromatograms were incubated with monoclonal (B) anti-sialyl-Lewis x, (C) anti-sialyl-Lewis a, and radiolabelled *H. pylori* (D) strain CCUG 17875, (E) the BabA1A2 mutant strain, and (F) strain CCUG 17874. Autoradiography was for 12 hr. The *lanes* contained: 1, Acid glycosphingolipids of calf brain, 40 µg; 2, Acid glycosphingolipids of human neutrophil granulocytes, 40 µg; 3, Acid glycosphingolipids of human neutrophil granulocytes after de-sialylation, 40 µg; 4, Acid glycosphingolipids of human gall bladder adenocarcinoma, 40 µg; 5, Acid glycosphingolipids of human gall bladder adenocarcinoma after de-sialylation, 40 µg; 6, NeuAcα3Galβ4(Fucα3)GlcNAcβ3Galβ4(Fucα3)GlcNAcβ3Galβ4Glcβ1Cer isolated from human gall bladder adenocarcinoma, 1 µg; 7,
NeuAcα3Galβ3(Fucα4)GlcNAcβ3Galβ4Glcβ1-Cer (sialyl-Lewis a hexaglycosylceramide) isolated from human gall bladder adenocarcinoma, 4 µg; 8,
NeuAcα3Galβ4(Fucα3)GlcNAcβ3Galβ4Glcβ1Cer (sialyl-Lewis x hexaglycosyl-ceramide), 4 µg; 9, Fucα2Galβ3(Fucα4)GlcNAcβ3Galβ4Glcβ1Cer (Lewis b-6 hexaglycosylceramide) of human small intestine, 4 µg.

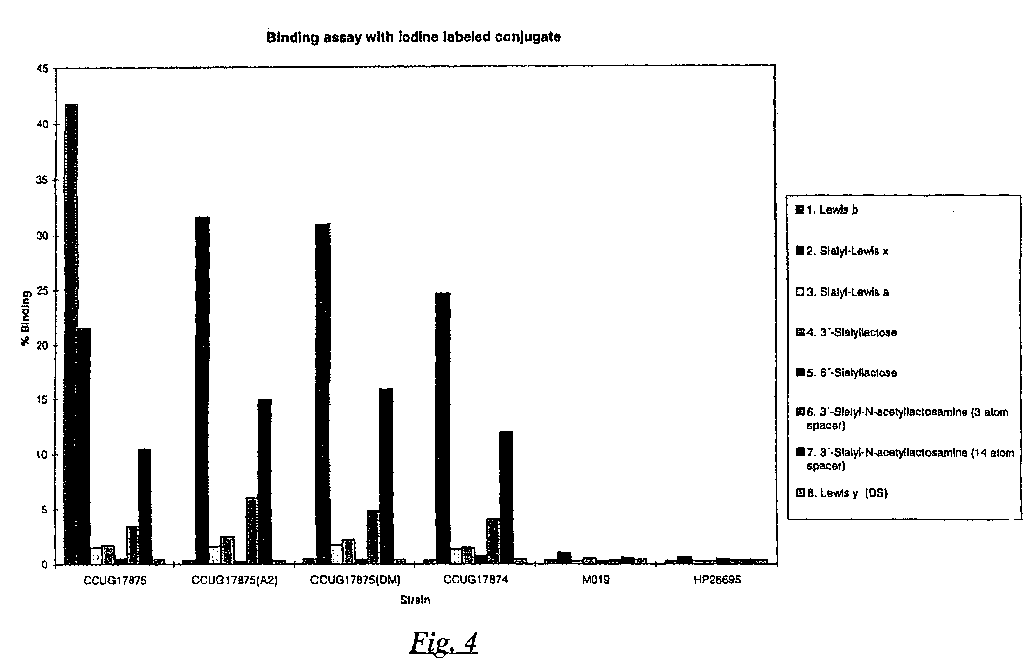

**Fig. 4** shows characterisation of *H. pylori* receptor specificity with soluble sialylated conjugates: *H. pylori* strains (see Bacterial Strains and Growth Conditions) were incubated with ¹²⁵I-labelled neo-glyco conjugates (See Experimental protocol: Radio Immuno analyses with neoglycoconjugate receptors). Bars, from the left to the right; The sialyl-alfa2.61actose-, sialyl-alfa2.31actose-, sialyl-Lewis a-, sialyl-Lewis x-, and Lewis b-, synthesised sialyl-Lewis x- sialyl-acetyllactosaminee, both with a either a 3 carbon or 14 carbon (extended/hydrophilic) spacer, conjugated to albumin, Finally, the histo-blood group antigen Lewis y (synthesised penta-saccharide) (V. Behar and S.J. Danishefsky, *Angew. Chem. Int. Ed. Engi,* 1994, 33, no. 14, 1468.), was analysed as a neo-glycoconjugate.

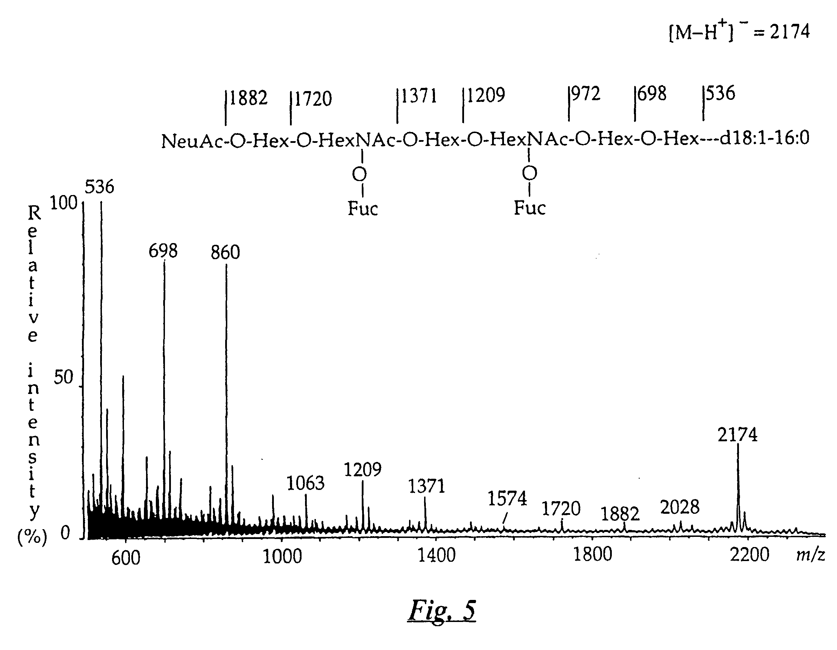

**Fig. 5** shows a negative ion FAB mass spectrum of the *H. pylori*-binding ganglioside isolated from human gall bladder carcinoma: The molecular ion (M-H⁺)⁻ at *m*/*z* 2174 indicates a glycosphingolipid with one NeuAc, two fucoses, two *N*-acetylhexosamines, four hexoses and d18:1-16:0. A series of fragment ions from *m*/*z* 2174, obtained by successive elimination of terminal carbohydrate units, are found at *m*/*z* 2028 (M-Fuc-H⁺)⁻, *m*/*z* 1882 (M-NeuAc-H⁺)⁻, *m*/*z* 1720 (M-NeuAc-Hex-H⁺)⁻, *m*/*z* 1574 (M-NeuAc-Hex-Fuc-H⁺)⁻, *m*/*z* 1371 (M-NeuAc-Hex-Fuc-HexNAc-H⁺)⁻, *m*/*z* 1209 (M-NeuAc-Hex-Fuc-HexNAc-Hex-H⁺)⁻, *m*/*z* 1063 (M-NeuAc-Hex-Fuc-HexNAc-Hex-Fuc-H⁺)⁻, *m*/*z* 860 (M-NeuAc-Hex-Fuc-HexNAc-Hex-Fuc-HexNAc-H⁺)⁻, *m*/*z* 698 (M-NeuAc-Hex-Fuc-HexNAc-Hex-Fuc-HexNAc-Hex-H⁺)⁻, and *m*/*z* 536 (M-NeuAc-Hex-Fuc-HexNAc-Hex-Fuc-HexNAc-Hex-Hex-H⁺)⁻. Thus the glycosphingolipid had a NeuAcHex(Fuc)HexNAcHex(Fuc)HexNAcHexHex sequence, as shown in the formula above the spectrum.

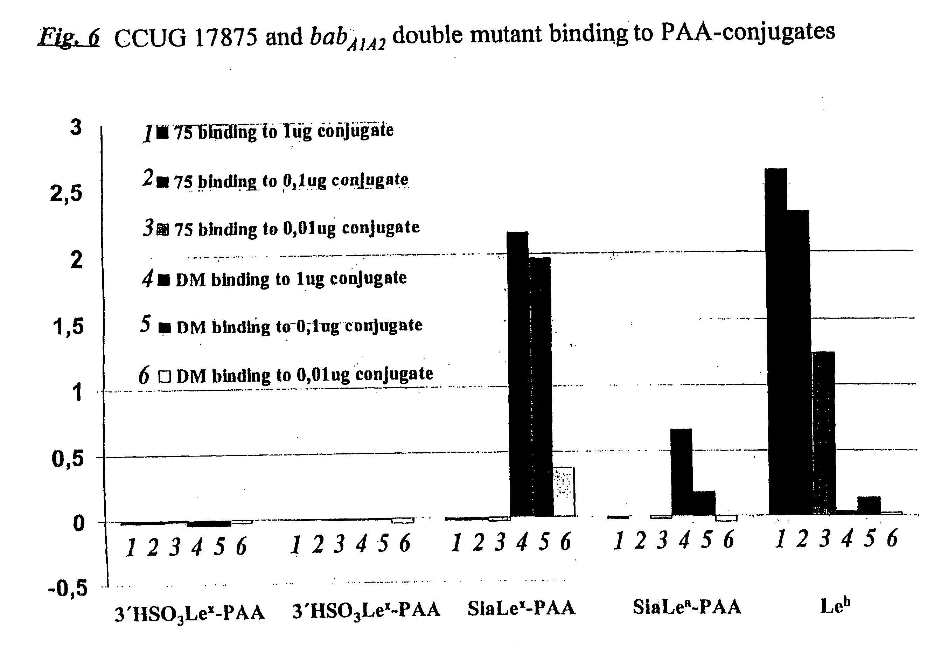

**Fig. 6** shows *H. pylori* adherence to immobilized neoglycoconjugates in ELISA: Sialyl-Lewis a-PAA, sialyl-Lewis x-PAA, 3'-HSO3-Lewis a-PAA, and 3'-HSO3-Lewis x-PAA, i.e. synthesised sialylated or sulphated Lewis antigens conjugated to polyacrylamide backbone were added in dilution series of 1µg, 100ng, and 10 ng, to streptavidin coated microtiter plats (ELISA). CCUG 17875 (75) and the *babA1A*_{*2*} double mutant (DM) were added to the ELISA in a series labelled A-F. Adherent bacteria were detected by polyclonal rabbit antisera against *H. pylori* CCUG 17875. The products of the horseradish peroxidase reaction were quantified in a microtiter plate reader.

Fig. 7A, B, C See Fig. 1.and Fig. 2

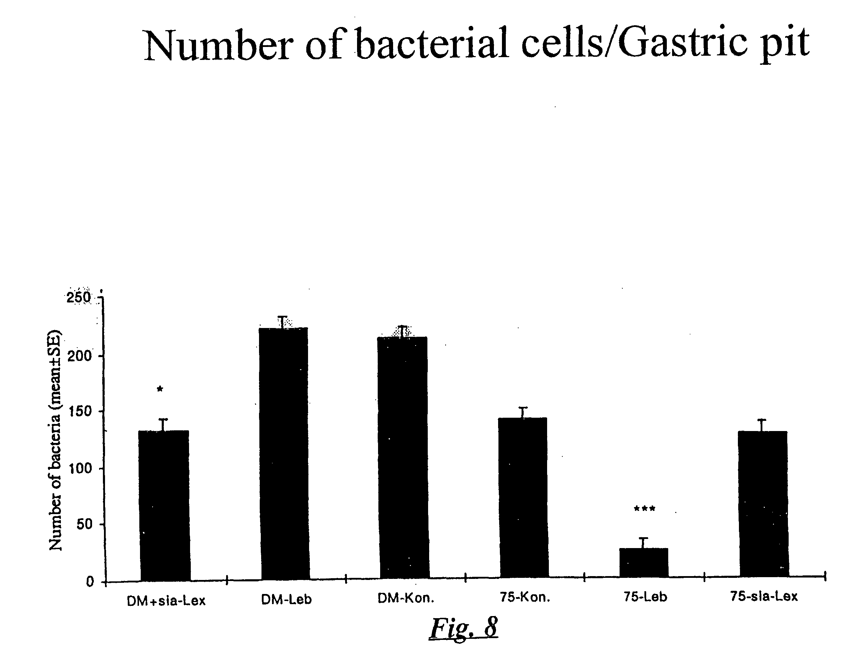

**Fig. 8** Antibody inhibitions of bacterial binding *in situ. H. pylori* CCUG 17875 (75) and the *babA1A2*-mutant (DM) were overlaid on tissue sections pre-treated with monoclonal antibodies against the Lewis b antigen (Lab)or the sialyl-Lewis x ( sia-Lex)antigen, and analyzed by *in situ* adherence. Each value is the mean number of adherent bacteria + -SEM of 10 different fields. A comparison between the two different bacterial strains (CCUG17875 *babA1*/*babA2*-mutant) and control (DM-kon, 75-kon) was performed with the Wilcoxon/Student T-test non-parametric test. P values below 0,05 were considered significant.

The following tables are attached to the description:
**Table 1**, which shows a summary of the results generated in Fig. 3;
**Table 2,** which shows a summary of results from binding of Helicobacter pylori to glycosphingolipids on thin-layer chromatograms, and
**Table 3,** which presents the glycoconjugates used in the present study.

### Description

The present inventors have now surprisingly identified a new host tissue carbohydrate receptor, mapped the receptor specificity and affinity and, in addition, found the presence of the new receptor to be highly correlated with the level of tissue-inflammation, suggesting an up-regulation of the new *H. pylori* receptor in infected and inflamed gastrointestinal mucosa. Consequently the use of a specific carbohydrate structure is disclosed. In this context, conditions involving gastrointestinal infection by *H. pylori* comprise chronic active gastritis, gastric ulcers, duodenal ulcers, gastric adenocarcinoma, and gastric lymphoma.

In order to characterize and identify the microbial part of the interaction, i.e. the corresponding histo-blood group antigen binding *H. pylori* adhesin, i.e. the BabA anhesin, the group combined the technique of affinity purification and crosslinker attachment, and developed a novel technique; Receptor Activity Directed Affinity Tagging (ReTagging), allowing the identification and purification of adhesins, (and subsequent cloning/sequencing) by the biological activity of the Lewis b antigen receptor (D. Ilver and T. Borén et al., *Science* 279, 373, 1998). In order to verify the correct identity of the cloned *babA*-adhesin gene, a babA-knock out mutant, i.e. a mutant lacking the babA adhesin gene(s) was constructed. This mutant subsequently lost the binding activity for the Lewis b antigen receptor, and the correct identity of the cloned babA-adhesin gene could be verified. The experiment was vital since the BabA adhesin belongs to a family of related and similar outer membrane genes and, in addition, two separate, but identical alleles of the babA adhesin gene were found (although only one allele was found to be expressed as a functional adhesin protein). These results were also published in: Ilver/Borén et al., *Science* 279, 373, 1998. It is proposed that specific receptor-adhesin mediated *H. pylori* adherence to gastric epithelial tissue plays a critical role in efficient delivery of *H. pylori* virulence factors that damage host tissue directly, and that incite inflammatory responses and provoke auto-immune reactions, that cumulatively lead to development of ulcer disease

The babA knock out mutant described above, lacking the Lewis b antigen binding properties, turned out to be extremely interesting because the mutant still binds avidly to the human gastric epithelial lining, in adherence experiments *in situ*, i.e. to histo-tissue sections of human (biopsy) gastric mucosa. These results suggest the activation of a complementary adhesin-receptor interaction, for optimal tissue targeting.

Consequently, the present inventors disclose the use of fucosylated sialylated N-acetyl lactosamine carbohydrate structures, such as sialyl-Lewis antigen carbohydrate structures for the preparation of a pharmaceutical composition for the treatment or prophylaxis in humans of conditions involving infection by *Helicobacter pylori* and related pathogens of the human gastric mucosa. In particular, said use in which the sialyl-Lewis antigen carbohydrate structure is capable of binding to adhesins present on the surface of *H. pylori*.

Fucosylated sialylated N-acetyl lactosamine structures are created by expression of alfa1,3fucosyltransferase activities capable of modifying acceptors containing alfa(2,3) sialic acid-substituted lactosamineoglycans, such as Lewis x, Galβ1.4(Fucalfa1.3)GlcNAcβ1-R (where R is a protein or other carbohydrate structure). The Lewis x antigen is a common trisaccharide structure, which forms the core of this sialylated structure, resulting in sialyl-Lewis x, i.e. NeuAcalfa2.3Ga.β1.4(Fucalfal)GlcNAc-R. Another possible structure would be difucosylated sialyl Lewis x, a longer polyfucosylated polylactosamineoglycan, or similar derivatives. It is suggested, that the number of fucose-residues in the core chain affects the affinity and aids in the stabilisation of the receptor-ligand complex/interaction. This interest was further increased by the finding that a binding pattern almost parallel to the pattern of strains CCUG 17874 and the mutant strains was obtained with monoclonal antibodies directed against sialyl-Lewis x (Fig 3, B). The optimal receptor could be of more complex type, as suggested by the slower migrating glycolipid bands that demonstrate high affinity receptor properties in the HPTLC-overlay analyses (Lanes 2 and 4).

A *H. pylori*-binding and anti-sialyl-Lewis x-reactive glycosphingolipid was isolated from the human gall bladder adenocarcinoma (Fig. 3, lane 6), and characterized by negative ion FAB mass spectrometry and ¹H NMR as NeuAcHex(Fuc)HexNAcHex-(Fuc)HexNAcHexHex with sphingosine and non-hydroxy 16:0 fatty acid (Fig. 5). Taken together with the anti-sialyl-Lewis x-binding activity a NeuAcα3Galβ4(Fucα3)-GlcNAcβ3Galβ4(Fucα3)GlcNAcβ3Galβ4Glcβ1Cer compound was suggested, *i.e.* a ganglioside with repetitive Lewis x core, previously described in human colonic adenocarcinoma (Fukushi, Y., Nudelman, E., Levery, S.B., Hakomori, S.-i., and Rauvala, H. (1984). Novel fucolipids accumulating in human adenocarcinoma. III. A hybridoma antibody (FH6) defining a human cancer-associated difucoganglioside (VI³NeuAcV³III³Fuc₂nLc₆). J. Biol. Chem. *259,* 10511-10517). The binding-active compound was thus a ganglioside with repetitive Lewis x core (Fig. 5).

While almost no binding to sialyl-Lewis a hexaglycosylceramide (Fig. 3, lane 7) was obtained, a weak binding of the CCUG 17874 strain, the BabA2 mutant and the BabA1A2 mutant to sialyl-Lewis x hexaglycosylceramide (lane 8) was occasionally observed. However, this binding required 2 nmol, while the detection level for NeuAcα3Galβ4(Fucα3)-GlcNAcβ3Galβ4(Fucα3)GlcNAcβ3Galβ4Glcβ1Cer was approximately 1 pmol.

The distinct difference in affinity argue for that the monomeric sialyl-Lewis x ganglioside exhibit features required for bacterial binding, while the dimeric or repetitive form is optimised in its presentation of the terminal sialic acid epitope.

At this stage an open question if the increased binding activity is dependent on the repetitive Lewis x element, or due to the longer carbohydrate chain giving optimal exposure of the binding epitope, as has been described for monoclonal anti-sialyl-Lewis x antibodies (Müthing, J., Spanbroek, R., Peter-Katalinic, J., Hanisch, F.-G., Hanski, C., Hasegawa, A, Unland, F., Lehmann, J., Tschesche, H., and Egge, H. (1996). Isolation and structural characterisation of fucosylated gangliosides with linear poly-N-acetyllactosamineyl chains from human granulocytes. *Glycobiology 6*, 147-156).

The sialyl-Lewis antigen carbohydrate structure is an antigen chosen among sialyl-Lewis x and sialyl-Lewis a and structurally related carbohydrates. According to a preferred embodiment, the sialyl-Lewis antigen carbohydrate structure is a dimeric or repetitive sialyl-Lewis antigen carbohydrate structure, for example an antigen chosen among dimeric sialyl-Lewis x and sialyl-Lewis a and in particular a repetitive sialyl-Lewis x type structure.

According to another embodiment, exemplified in the attached examples, the bacterial adherence can be influenced by antibodies, raised against the sialyl-Lewis structure. The manufacture of suitable antibodies, either monoclonal or polyclonal antibodies, is well known to persons skilled in the art.

A pharmaceutical composition comprising a sialyl-Lewis antigen or corresponding antibody as the main active substance or as one of several active substances can be prepared in a conventional manner, equal to the preparation of other compositions for administration to the gastrointestinal tract. Suitable pharmaceutical vehicles for administration to a patient are known to those skilled in the art.

Said pharmaceutical composition may preferably be administered orally, parenterally or per rectum. It is conceivable that the pharmaceutical composition is given to the patient in drinkable form, comprising suitable constituents such as starches, sugars, fats and waxes and may further include flavouring agents, colouring agents, disintegrants, binders, dispersants etc. It is also conceivable that the pharmaceutical composition is delivered locally to the stomach or duodenum via a stomach tube or nasogastric or nasoduodenal tube. Suitable administration forms include liquid, e.g. aqueous suspensions, as well as solid forms, such as tablets, capsules or suppositories.

For parenteral administration, the sialyl-Lewis x type carbohydrate structure or corresponding antibody can be dissolved or suspended in sterile water or physiological saline solution. For enteral administration, the carbohydrate can be incorporated into an inert carrier and processed to a tablet, capsule or similar form.

Alternatively, the carbohydrate or corresponding antibody can be administered in liposomes or microspheres / microparticles. Methods for preparing liposomes and microspheres for administration to a patient are known to those skilled in the art, and these can be tailored according to need, for example to obtain delayed release or release at a specific location in the gastrointestinal tract, dependent of pH, temperature or the like.

The dosage of the sialyl-Lewis antigen or corresponding antibody depends on the choice of administration route, the particular condition to be treated and the severity of the same, and also whether the disease is to be treated or prevented, as well as the age and weight of the patient to be treated. Finding the optimal dose in each case lies within the normal skills of a practitioner in the field. Importantly, the carbohydrates should be active when administered parenterally or by other means. The amounts needed will be based on the concentrations required for inhibition *of H. pylori* cells in *in vitro* assays and the clearance rates of the carbohydrates. The dosage is also dependent on whether one or more carbohydrates are administered. A synergistic effect may be seen with combinations of carbohydrates, other pharmaceuticals such as antibiotics, multivalent forms of the natural ligand or derivatives thereof, designed to increase affinity and/or avidity for *H. pylori*.

The present invention will be exemplified in the following, examples:

### Examples

### Experimental procedures

### Bacterial Strains and Growth Conditions

*H. pylori* strain CCUG17875 and CCUG 17874 (Australian) were obtained from CCUG, Göteborg, Sweden. Strain MO19 (US) and was described previously (T. Borén, P. Falk, K. A. Roth, G. Larson, and S. Normark, *Science*. **262,** 1892 (1993),). Strain 26695 was recently genomically sequenced (J-F. Tomb et al., *Nature* **388,** 539 (1997).). The molecular construction and Lewis b antigen binding properties of the *babA2*-mutant was recently described (D. liver, et al., *Science,* 279, 373 (**1998**)). The panel of 91 *H. pylori* fresh clinical isolates came from the University Hospital in Uppsala, Sweden. Bacteria were grown at 37°C in 10 % CO₂ and 5% O₂ (T. Borén, P. Falk, K. A. Roth, G. Larson, and S. Normark, *Science..* 262, 1892(1993), for 2 days for optimal Le b antigen binding activity.

### Bacterial in situ adhesion and inhibition assay

Bacterial in situ adherence assay (Fig. 1 and Fig 2) was as described (P. Falk *et al., Proc. Natl. Acad. Sci. U.S.A*., **90**, 2035 (1993)). Human stomach samples were obtained from the Department of Gastroenterology, Barnes-Jewish Hospital/Washington University, St. Louis. Bacteria were labelled with fluorescein isothiocyanate (FITC), Sigma, St. Louis. Bacterial suspensions were diluted to 0.15 OD₆₀₀ in blocking buffer and 200µL was applied to the sections, incubated for 1h at room temperature, and washed 6x5min (also described in Falk, P., Borén, T., Haslam, D., Caparon M.G. (1994). Bacterial Adhesion and Tissue Colonization Assays *Methods. Cell Biology,* 45, 165-192; Borén T., Wadström T., Normark S., Gordon. J. I., and. Falk P. G. (1997) Methods for the Identification of H. *pylori* Host Receptors. *Meth. Molecular Medicine, 8, 205-224.* The ability of glycoconjugates to inhibit the bacterial adherence to human stomach in situ was analysed with Lewis b conjugates in 10µg/mL concentration, and the sialyl-Lewis conjugate used in 20µg/mL concentration. Reduction in bacterial binding was estimated by counting the number of adherent bacteria under 200X magnification (Fig. 7 A, B, C). Each value is the mean +- SEM of 10 different fields. The control where bacteria were not pre-incubated with glycoconjugates was defined as 100% binding.
Statistical Analysis of bacterial adherence in situ and inhibition experiments; A comparison between the two different bacterial strains (CCUG 17875 and double mutant (DM)) and control was performed with the Wilcoxon/Student T-test non-parametric test. P values below 0,05 were considered significant. Value £ 0,001(***); Value £ 0,01 (**); Value < 0,05 (*)

### Preparation, purification and identification of glycosphingolipid receptors

Glycosphingolipids were isolated and characterized by mass spectrometry, ¹H NMR, and degradation studies, as described (Karlsson, K.-A. (1987). Preparation of total non-acid glycolipids for overlay analysis of receptors for bacteria and viruses and for other studies. Methods Enzymol. *138*, 212-220. A *H. pylori*-binding glycosphingolipid was isolated from human gall bladder adenocarcinoma by repeated chromatography on silicic acid columns of the native glycosphingolipid fractions, or acetylated derivatives thereof. Mild acid hydrolysis was done by incubating the glycosphingolipids in 1% (v/v) acetic acid for 1 hr at 100°C.

Negative ion FAB mass spectra were recorded on a JEOL SX-102A mass spectrometer (JEOL, Tokyo, Japan). The ions were produced by 6 keV xenon atom bombardment, using triethanolamine as matrix and an accelerating voltage of -10 kV.

### Analyzes of H. pylori binding to separated glycosphingolipids

Culture and ³⁵S-labelling of *H. pylori* was as outlined in (Ångström, J., Teneberg, S., Abul Milh, M,. Larsson, T., Leonardsson, I., Olsson, B.-M., Ölwegård Halvarsson, M., Danielsson, D., and Karlsson, K.-A. (1998). The lactosylceramide binding specificity of *Helicobacter pylori*. Glycobiology *8*, 297-309.). Mixtures of glycosphingolipids (20-40 µg/lane) or pure compounds (0.002-4 µg/lane) were separated on aluminium-backed silica gel 60 HPTLC plates (Merck), using chloroform:methanol:water 60:35:8 (v/v/v) as solvent system. Chemical detection was done with anisaldehyde (Waldi, D. (1962) Sprühreagentien für die dünnschicht-chromatographie. In Dünnschicht-Chromatographie, E. Stahl ed. (Berlin: Springer-Verlag) pp. 496-51).

Binding of radiolabelled *H. pylori* to glycosphingolipids on thin-layer chromatograms was performed as described above (Ångström et al., 1998) using suspensions of bacteria diluted in phosphate-buffered saline (PBS), pH 7.3, to 1x10⁸ CFU/ml. The specific activities of the suspensions were approximately 1 CPM per 100 *H. pylori* organisms. Binding of monoclonal antibodies was done as described (Magnani, J.L., Brockhaus, M., Smith, D.F., Ginsburg, V., Blaszczyk, M., Mitchell, K.F., Steplewski, Z., and Koprowski, H. (1981). A monosialoganglioside is a monoclonal antibody-defined antigen of colon-carcinoma. Science 212, 55-56). Anti-sialyl-Lewis x monoclonal antibodies (clone KM-93) were from Seikagaku Corp., Tokyo, Japan and anti-sialyl-Lewis a monoclonal antibodies (CA 19-9) from Signet Laboratories, Inc., Dedham, MA 02026, USA.

### Radio Immuno Analyses with Neoglycoconjugate Receptors

The sialyl-alfa2.3lactose-, sialyl-alfa2.61actose-, sialyl-Lewis a-, and Lewis b-oligosaccharide antigens used for neoglycoconjugate preparations were purified by HPLC, structurally identified and characterized with NMR-spectroscopy, and more than 95% pure. These conjugates were attached to albumin with an APD (acetylphenylenediamine) spacer attached to the carbohydrate by reductive animation and thus the terminal reducing monosaccharide unit of the oligosaccharide is reduced and is present as an aminoalditol, IsoSep AB, Tullinge, Sweden (T. Borén, P. Falk, K. A. Roth, G. Larson, and S. Normark, *Science.* **262**, 1892 (1993), P. D. Rye, *Nature Biotechnology* **2**, 155 (1996)). The synthesised sialyl-Lewis x- tetrasaccharide antigen was conjugated to albumin with the MPE (mercaptopropionyl-amidoethyl) -spacer, IsoSep AB. Synthesised sialyl-Lewis x-tetrasaccharide and sialyl-acetyllactosaminee, both with a either a 3 carbon or 14 carbon (extended/hydrophilic) spacer, conjugated to albumin, were obtained from Dextra Laboratories, Reading, UK. The RIA (Fig. 4) was performed according to (P. Falk, T. Borén, D. Haslam, M. G. Caparon, *Meth. Cell Biol.* **45**, 161 (1994)) with minor modifications; the glycoconjugates were ¹²⁵I-labelled by the Chloramine T method. 1 ml of bacteria (A₆₀₀= OD 0.10) was incubated with 400 ng of ¹²⁵I-labelled conjugate (that is an excess of receptor substrate) for 30 min. in phosphate buffered saline (PBS), 0.5 % albumin, 0.05 % Tween-20 (BB-buffer). After centrifugation, the ¹²⁵I-activity bound to the bacterial pellet was measured by gamma scintillation counting. Binding experiments were reproducible and performed in triplets. In addition, the level of binding-activity within the strain was stabile.

### Construction of the bab-mutants

To make the *babA* deletions, *babA* including the *baba2* upstream sequence was amplified by the F2 (forward) and R39 (reverse) primers and cloned in pBluescript SK (Stratagene, La Jolla). The vector was linearised withR41 and F38. The camR gene (Y. Wang and D. E. Taylor, *Gene* **94**, 23 (1990)) was ligated between the fragments, and strainCCUG17875 was transformed with selection for CamR. The *H. pylori* transformants were analyzed for binding to ¹²⁵I-labelled Le b glycoconjugate and the location of the camR gene, and thus the copy mutated, was analyzed by PCR with the upstream primers F2 (*babA2*) or F44 (*babA1*) in combination with primer R11.

By introduction of the inactivated babAl-allele into the *babA2*-mutant, the *babA1A2*-double mutant was constructed. In order to select for both inactivated alleles, a second resistance marker KanR was introduced into the second babA-allele.

The used primers had the following sequences:

F2:CTTAAATATCTCCCTATCCC; R39: CAAATACACGCTATAGAGCC;

R41: GCGAGCCTAAAGTTAATGA; F38: ACGTGGCGAACTTCCAATTC;

F44: CAGTCAAGCCCAAAGCTATGC; R11: CGATTTGATAGCCTACGCTTGTG

### H. pylori adherence to immobilized neoglycoconjugates in ELISA (Fig. 6)

Sialyl-Lewis a-PAA, sialyl-Lewis x-PAA, 3'-HSO3-Lewis a-PAA, and 3'-HSO3-Lewis x-PAA, i.e. synthesised sialylated or sulphated Lewis antigens conjugated to polyacrylamide backbone were obtained from Syntesome GmbH, Munchen, Germany. The conjugates were added in dilution series of 1µg, 100ng, and 10 ng, in 90µl 1% BSA PBS 0.05% Tween, to streptavidin coated microtiter (ELISA) plates (Roche Diagnostics Scandinavia AB, Bromma, Sweden). After one hour of incubation in room temperature, the plate was blocked over night at 4°C, with 500ng biotinylated albumin in 300µl 1% BSA PBS 0.005% Tween. CCUG 17875 and the *babA1A*_{*2*} double mutant were harvested after 43 hours incubation on blood agar plates (see Bacterial Strains and Growth Conditions), washed once in 1% BSA in PBS 0.05% Tween, and then resuspended in 1% BSA in PBS 0.05% Tween. A final OD₆₀₀ was set to 0.5/ ml (**5*10**^{**6**} CFU/ ml). 150 µl bacteria was added to each well. Plates were incubated for 1 hour in room temperature and then washed three times in 300µl PBS 0.05% Tween. Adherent bacteria were detected by polyclonal rabbit antisera at a dilution of 1:3000, raised against formalin fixed bacteria of strain CCUG 17875 in New Zealand rabbits (Agrisera AB, Vännäsby, Sweden) The antibodies were allowed to incubate in 1% BSA in PBS 0.05% Tween for 30 minutes at room temperature. 150 µl of the suspension was added to the wells for 1 hour at room temperature The same procedure were performed for the secondary anti rabbit antibody conjugated to HRP (DAKO, Goat anti rabbit immunogloubulins-HRP) at a dilution of 1:1000 after three washes in 300µl PBS 0.05% Tween. After three final washes in 300µl PBS 0.05% Tween, 100 µl peroxidase substrate (3.5 mM H₂O₂ and 2.2 mM *o*-phenylene diamine hydrochloride in 0.05 M sodium citrate phosphatase buffer, pH 5.0) was added. After 5 minutes the reaction was stopped by adding 25 µl 4 M H₂SO₄. The products of the horseradish peroxidase reaction were quantified in a microtiter plate reader at 490 nm.

### Antibody inhibitions of bacterial binding in situ.

*H. pylori* CCUG 17875 and the *babA1A2-* -mutant were overlaid on tissue sections pre-treated with monoclonal antibodies and analyzed by *in situ* adherence. Gastric tissue sections were pre-incubated with anti-Lewis b monoclonal antibodies, Immucor, Inc. Norcross, GA or anti-sialyl-Lewis x monoclonal antibodies (clone KM-93), Seikagaku Corp., Tokyo, Japan, in a dilution of 1:100x for one hour. *H. pylori* was with an ODA600=0,2 was overlaid as described in (described (P. Falk *et al., Proc. Natl. Acad. Sci. U.S.A*., **90**, 2035 (1993)). Excess bacterial cells were removed by 6x5 min washings in (BB)-blocking buffer.. Reduction in bacterial binding was estimated by counting the number of adherent bacteria under 200X magnification (Fig. 8). Each value is the mean +- SEM of 10 different fields. The control where bacteria were not pre-incubated with glycoconjugates was defined as 100% binding. Statistical Analysis of bacterial adherence in situ and inhibition experiments; A comparison between the two different bacterial strains (CCUG17875 and *babA1A2*-double mutant (DM)) and control was performed with the Wilcoxon/Student T-test non-parametric test. P values below 0,05 were considered significant.
Value £ 0,001 (***); Value £ 0,01 (**); Value < 0,05 (*)

### Results

### 1. The H. pylori babA-mutant adheres to the gastric tissue independent of the Lewis b antigen

babA-mutants in strain CCUG 17875 had previously been made by inactivation of the babA1 allele, without phenotypic effect, or by inactivation of the *babA2* allele, with concomitant loss of the Lewis b antigen binding properties. The latter results supported the results of the BabA-protein as the Lewis b antigen binding adhesin (D. liver et al., *Science* 279, 373 (1998)). In the present series of experiments, the *babA2* mutant was analyzed for binding properties by the *in situ* adherence assay. The binding activity in situ was found to be close to 50% compared to the CCUG 17875 wild-type strain (by the number of adherent bacterial cells),and the binding pattern of the mutant looks most similar to the binding pattern of the *H. pylori* CCUG 17875 wild-type (wt) bacteria (data not shown). Since recombination in between babA alleles could possible have moved the silent babA1 allele into thebabA2-allele, (by replacement of the CamR cassette), a *babA1*/*babA2*-(a KanR/CamR)-mutant was constructed, where both babA-alleles were inactivated by recombination. However, the binding activity *in situ* by the *babA1*/*babA2*-mutant was still found to be close to 50% compared to the CCUG 17875 wild-type strain (Fig. 7A, by the number of adherent bacterial cells) and the binding pattern of the *babA1*/*babA2*-mutant looks most similar to the binding pattern of the *H. pylori* CCUG 17875 wild-type (wt) bacteria (Fig. 1 B, C). Binding to the surface mucus cells was previously shown to be mediated by the fucosylated histo-blood group antigens, such as the Lewis b antigen (T. Borén et al. *Science 1993*, 262, 1892). Pre-incubation of the *H. pylori* wt-strain with low concentrations (10µg/mL) of soluble Lewis b antigen (IsoSep AB, Tullinge, Sweden) results in an almost total inhibition (80% reduction) in binding (Fig. 1E and Fig. 7A). In contrast, binding by the *babA1*/*babA2*-mutant to the surface mucus cells was not possible to inhibit by soluble Lewis b antigen (Fig. 1D), suggesting that the *ba babA1*/*babA2*-mutant strain has induced the expression of a complementary binding/adhesive property, and subsequent recognition of a distinctly different receptor.

### 2. Adherence of the H. pylori babA1/babA2-mutant is dependent on expression of alfa1,3/4 fucosyl transferase in the gastric mucosa

Adherence experiments *in situ* by *H. pylori* wild type and the *babA1*/*babA2*-mutant were also performed using histo-tissue sections of mouse (FVB/N) and, in addition, the transgenic "Lewis b mouse" gastric mucosa. The "Lewis b mouse" express the alfa 1,3/4 fucosyl transferase (alfa1,3/4 FT) in the mouse gastric mucosa, resulting in the expression of the (otherwise) human specific Lewis b antigen in the pit and surface mucus cells (P.G. Falk et al., (1995) *PNAS*, **92**, 1515). In analogy with previous data (P.G. Falk et al, (1995) *PNAS,* **92**, 1515). the *H. pylori* wt-bacteria bind poorly to normal mouse gastric mucosa *in situ,* while binding is efficient to the "Lewis b mouse" gastric mucosa, i.e. to the surface mucus cells (**Fig 2 C, E**). The new results demonstrate that the *babA1*/*babA2*-mutant bacteria behave very similar, i.e. there is an efficient binding activity induced by the healthy "Lewis b mouse" gastric mucosa i.e. to the surface mucus cells (Fig. 2 D). In contrast, binding by the *babA1*/*babA2*-mutant to non-transgenic mouse is poor and limited to the most upper layer, most likely to the mucins in the protective mucus (**Fig 2 B**). These results suggest that the receptor utilised by the *babA1*/*babA2*-mutant is dependent of the additional alfa-1.3/4-fucose residue, added by the power of the human-specific fucosyl-transferase activity of the "Lewis b mouse".

### 3. The babA1/babA2-mutant demonstrate affinity for human specific sialylated glycosphingolipids, i.e., for gangliosides

Screening of potential receptor structures unique for *thebabA1*/*babA2*-mutant was performed by adherence experiments to well-defined panels of MS-characterized glycolipids, from defined and unique sources and tissues. Binding of *H. pylori* and monoclonal antibodies to glycosphingo-lipids was evaluated using the thin-layer chromatogram binding assay (J. Ångström *et al., Glycobiology* **8,** 297 (1998); J. M. McKibbin *et al., J. Biol. Chem.* **257**,755 (1982)), i.e., glyco-lipids were separated on HPTLC-plates, and H. *pylori* CCUG 17875 wild type bacteria and the *babA1*/*babA2*-mutant were overlaid on the plates and binding of radio labelled bacterial cells was detected. (See Experimental procedures). Four H. *pylori* strains were tested; CCUG 17874 which binds to glycosphingolipids in a sialic acid-dependent manner, but does not recognize the Le^{b} epitope (D. Ilver et al., Science 279, 373 (1998); Miller-Podraza, H., Abul Milh, M., Teneberg, S., and Karlsson, K.-A. (1997) Binding of *Helicobacter pylori* to sialic acid-containing glycolipids of various origins separated on thin-layer chromatograms. Infect. Immun. 65, 2480- 2482), the Lewis b-binding strain CCUG 17875, which is devoid of sialic acid binding capacity (liver et al., 1998; Miller-Podraza et al., 1997), the *babA2*-mutant and the *babA1A2*-mutant. The results from Fig. 3 are summarised in Table 1.

The *babA2* mutant and the *babA1A2* mutant differed from the parent strain CCUG 17875 in two respects. Unlike the 17875 strain, the *babA2* mutant and the *babA1A2* mutant did not recognize the Lewis b-6 glycosphingolipid (Fig. 3E, lane 9), i.e. the expected behaviour of the *babA*-mutants. Instead, another binding capacity has emerged, manifested by the binding of these mutant strains to slow-migrating acid glycosphingolipids of human neutrophil granulocytes and human gall bladder adenocarcinoma (Fig. 3E, lanes 2 and 4) in a manner that was indistinguishable from the binding pattern obtained with strain CCUG 17874. The binding of the *babA2* mutant, the *babA1A2* mutant, and CCUG 17874 was abrogated by the removal of sialic acid by mild acid hydrolysis (See Experimental protocol) (Fig. 3, lanes 3 and 5), demonstrating the involvement of sialylated structures in the binding process. However, no binding to the ganglio-series gangliosides of calf brain was obtained (lane 1, and Table 1, Nos. 1-4), suggesting the involvement of additional structures for a full receptor epitope, since the presence of sialic acid *per se* was not enough to support bacterial binding.

In conclusion, specific binding by the *babA1A2* mutant was restricted to sialylated (acidic) glycolipids from human sources, such as from granulocytes. In contrast, there was no binding by the *H. pylori* wt-strain, and, in addition, no binding by the *babA1A2* mutant was detected after de-sialylation, or to sialylated glycolipids from non-human sources.

The gangliosides of human neutrophil granulocytes have been thoroughly characterized (Fukuda, M.N., Dell, A., Oates, J.E., Wu, P., Klock, J. C., and Fukuda, M. (1985). Structures of glycosphingolipids isolated from human granulocytes. J. Biol. Chem. *260,* 1067-1082; Stroud, M.R., Handa, K., Salyan, M.E.K., Ito, K., Levery, S.B., Hakomori, S.-i., Reinhold, B.B., and Reinhold, V.N. (1996a). Monosialogangliosides of human myelogenous leukemia HL60 cells and normal human leukocytes. 1. Separation of E-selectin binding from non-binding gangliosides, and absence of sialosyl-Lewis x having tetraosyl to octaosyl core. Biochemistry 35, 758-769; Stroud, M.R., Handa, K., Salyan, M.E.K., Ito, K., Levery, S.B., Hakomori, S.-i., Reinhold, B.B., and Reinhold, V.N. (1996b). Monosialogangliosides of human myelogenous leukemia HL60 cells and normal human leukocytes. 2. Characterisation of E-selectin binding fractions, and structural requirements for physiological binding to E-selectin. Biochemistry 35, 770-778; Müthing, J., Spanbroek, R., Peter-Katalinic, J., Hanisch, F.-G., Hanski, C., Hasegawa, A., Unland, F., Lehmann, J., Tschesche, H., and Egge, H. (1996). Isolation and structural characterisation of fucosylated gangliosides with linear poly-N-acetyllactosamineyl chains from human granulocytes. Glycobiology 6, 147-156). The main components are the GM3 ganglioside and sialylneolactotetraosylceramide. The larger gangliosides are based on repetitive N-acetyllactosaminee units, and in many cases carry Fuc(alfa)-residues linked to the 3-position of GlcNAc. In addition, NeuAc may be alfa3- or alfa6- linked to the terminal Gal of neolactotetraosylceramide and the longer glycosphingolipids with repetitive *N*-acetyllactosaminee units. Gangliosides with repetitive *N*-actyllactosaminee core substituted with fucose residues have also been described in human adenocarcinomas (Hakomori, S.-i. (1989) General concept of tumour-associated carbohydrate antigens: their chemical, physical and enzymatic basis. In Gangliosides and Cancer, H.F. Oettgen, ed. (New York: VCH Publishers) pp. 57-68).

The results from the *in situ* adherence- and inhibition- experiments, in combination with the HPTLC bacterial overlay assay, suggest the *babA1A2* mutant receptor to be sialylated, and in addition, dependent on alfa-1.3/4-fucose residue(s), i.e. much similar to the well-known selectin-receptors and tumour markers, the sialyl-Lewis x and sialyl-Lewis a-antigens (J. Sakamoto *et al., Cancer Res*., **49**, 745 (1989)., Takada *et al., Cancer Res.,* **53**, 354 (1993), Amado *et al., Gastroenterology*, 114, 462, 1998). However, in the HPTLC overlay experiments, almost no binding was detected to purified sialyl-Lewis a glycolipid.

### 4. The receptor specificity, affinity and prevalence of H. pylori for sialylated receptors

The detailed receptor specificity was analyzed by binding experiments to radio labelled semi-synthetic glycoproteins (i.e. neo-glyco-conjugates).These were either made from natural/purified or synthesised sialylated oligosaccharides conjugated to human serum albumin (**Fig. 4**). Interestingly, the binding experiments demonstrated a high binding capacity to sialyl-Lewis X for both the *BabA2* mutant, the *babA1A2* mutant and by the *H. pylori* CCUG 17875 and CCUG 17874 wild-type bacteria. Binding by the wild-type is reproducible approx. 30% reduced, compared to the mutant (Fig. 4), suggesting either a down regulation in expression of the corresponding adhesin or sterical hindrance in presentation or binding activity. In addition, alfa2.31actoseamine with a 14 carbon spacer linked to the albumin core demonstrated rather efficient binding, while the equivalent structure linked by a shorter, 3 carbon spacer, is much reduced in binding, The improved binding by the longer spacer, suggest the importance of steric flexibility in correct receptor presentation for optimal interaction with the bacterial adhesin. Poor binding was also demonstrated by the related sialyl-alfa2.31actose-, sialyl-alfa2.61actose-, and sialyl-Lewis a- antigens.

Nevertheless, the terminal alfa 2.3 linked sialic acid residue is crucial for receptor activity. Binding experiments with the closely related Lewis y pentasacharide antigen did not detect any binding activity (Fig. 4), pointing to the involvement of the sialylated terminal for correct formation of the receptor epitope.

In Fig. 4 several combinations of adherence properties are exemplified. The CCUG 17875 strain is subsequently competent for interaction with both the soluble Lewis b antigen and the sialyl-Lewis x antigen, while the CCUG 17874 strain (another clinical wild-type isolate and, in addition, type strain), is restricted to sialyl-Lewis x antigen binding, i.e. similar to the *babA2* mutant and the *babA1A2* mutant. Interestingly, both the MO19 strain and the HP 26695 strain (genomically sequenced by the TIGR-institute) (J-F. Tomb et al., *Nature* **388,** 539 (1997)) are devoid of both binding properties.

The earlier determination of affinity, reported in SE 9901007-6 by the same inventors involved a sialyl-Lewis X antigen less suited for Scatchard affinity analyses. The present inventors have therefor, during the priority year, analyzed the affinities of the *babA1A2*-mutant for sialylated conjugates according to Scatchard (see Table 2) (Scatchard, A.G. (1949) The attractions of proteins for small molecules and ions. Ann. N.Y. Acad. Sci. *51*, 660-672.). The *babA1A2*-mutant demonstrated the high affinity of 1x10⁸M⁻¹ for the sialyl-Lewis x antigen. The repetitive sialyl-Lewis x antigen was synthesized and confer the high affinity of 2x10⁸M⁻¹. The oligosaccharide part of the repetitive sialyl-Lewis x antigen conjugate was synthesized enzymatically from lactose, using the appropriate glycosyl transferases and nucleotide sugars (M Bårström, M Bengtsson, O Blixt, and T Norberg: "New lipophilic derivatives of reducing oligosaccharides and their use in enzymatic reactions: efficient synthesis of sialyl lewis a and sialyl dimeric lewis x glycoconjugates", Submitted to Carbohydrate Res., 2000).

Conjugation to Human Serum Albumin was carried out by the isothiocyanate procedure (E Kallin, H Lönn, T. Norberg: "New derivatization and separation procedures for reducing oligosaccharides", Glycoconjugate J. 3, (1986) 311-319). An average substitution degree of 11 haptens/HSA molecule was obtained, according to MALDI-TOF mass spectroscopy. In contrast, sialyl(alfa)2,31actose, i.e., the equivalent core structure devoid of fucose residues, demonstrate the 20-fold lower affinity of 1x10⁷M⁻¹. For the *babA1A2*-mutant, close to 1000 binding sites for the sialyl-Lewis x conjugate was found, i.e. similar to the number of binding sites for the Lewis b antigen receptor (liver, D., Arnqvist, A., Ögren, J., Frick, I.M., Kersulyte, D., Incecik, E.T., Berg, D.E., Covacci, A., Engstrand, L. and Boren, T. (1998) *Helicobacter pylori* adhesin binding fucosylated histo-blood group antigens revealed by retagging. Science 279, 373-377.).

In order to investigate the importance of the sialic acid residue for the formation of the bacterial binding site, the di-fucosylated Lewis y penta-saccharide antigen where the sialic acid residue is replaced with an alfa1,2fucose residue (Table 3) (Behar, V. and Danishefsky, S.J. (1994) A highly convergent synthesis of the Lewis y blood group determinant in conjugatable form, Angew. Chem. Int. Ed. Engl. *33*, 1468-1470), and the sialyl-alfa 2,6 lactose conjugate were analyzed, but did not demonstrate any binding activity (data not shown). The combined results point to the strict requirement for the alfa 2,3 linked sialic acid residue as a critical component in formation of the sialyl-Lewis x type of receptor determinant for *H. pylori* in combination with the repetitive Le x antigen.

The prevalence of sialyl-Lewis x antigen binding activity was assessed among 91 Swedish clinical *H. pylori* isolates and 44%, that is 40 isolates, bound the neoglycoconjugate antigen. None of the reference strains (Fig. 4A), nor any of the 91 clinical isolates, bound to the related Lewis y penta saccharide antigen (data not shown). These results provide evidence for the prevalence of the new sialyl-based Lewis x receptor also in natural clinical isolates. In addition, 14 out of 91 strains, i.e. 15%, demonstrate binding to the sialyl-Lewis a antigen. Interactions with the sialyl-Lewis a antigen is extremely intriguing considering sialyl-Lewis a as an established tumour antigen (especially in relation to *H. pylori* as a possible carcinogen). In case of cell transformation, and emergence of tumour markers such as sialyl-Lewis a, *H. pylori* might possibly have an additional receptors candidate available.

### 5. Preparation and identification of high affinity H. pylori ganglioside receptors

The interest of the present inventors was thus directed towards fucosylated neolactoseries gangliosides. Fucosylated sialylated lactosamine structures are created by expression of alfa1,3/(4) fucosyltransferase activities capable of modifying acceptors containing alfa(2,3) sialic acid-substituted lactosamineoglycans, such as Lewis x, Galβ1.4(Fucalfa1.3)-GlcNAcβ1-R (where R is a protein or other carbohydrate structure). The Lewis x antigen is a common trisaccharide structure, which forms the core of this sialylated structure, resulting in sialyl-Lewis x, i.e. NeuAcalfa2.3Ga.β1.4(Fucalfa1)GlcNAc-R. Another possible structure would be difucosylated sialyl Lewis x, a longer polyfucosylated polylactosamineoglycan, or similar derivatives. The number of fucose-residues in the core chain might affect the affinity and aid in the stabilisation of the receptor-ligand complex/interaction. This interest was further increased by the finding that a binding pattern almost parallel to the pattern of strains CCUG 17874 and the mutant strains was obtained with monoclonal antibodies directed against sialyl-Lewis x (Fig 3, B). The optimal receptor could be of more complex type, as suggested by the slower migrating glycolipid bands that demonstrate high affinity receptor properties in the HPTLC-overlay analyses (Lanes 2 and 4).

A *H. pylori*-binding and anti-sialyl-Lewis x-reactive glycosphingolipid was isolated from the human gall bladder adenocarcinoma (Fig. 3, lane 6), and characterized by negative ion FAB mass spectrometry and ¹H NMR as NeuAcHex(Fuc)HexNAcHex(Fuc)HexNAcHexHex with sphingosine and non-hydroxy 16:0 fatty acid (Fig. 5). Taken together with the anti-sialyl-Lewis x-binding activity a NeuAcα3Galβ4(Fucα3)GlcNAcβ3-Galβ4(Fucα3)GlcNAcβ3Galβ4Glcβ1Cer compound was suggested, *i.e.* a ganglioside with repetitive Lewis x core, previously described in human colonic adenocarcinoma (Fukushi, Y., Nudelman, E., Levery, S.B., Hakomori, S.-i., and Rauvala, H. (1984). Novel fucolipids accumulating in human adenocarcinoma. III. A hybridoma antibody (FH6) defining a human cancer-associated difucoganglioside (VI³NeuAcV³III³Fuc₂nLc₆). J. Biol. Chem. 259, 10511-10517). The binding-active compound was thus a ganglioside with repetitive Lewis x core (Fig. 5).

While almost no binding to sialyl-Lewis a hexaglycosylceramide (Fig. 3, lane 7) was obtained, a weak binding of the CCUG 17874 strain, the BabA2 mutant and the BabA1A2 mutant to sialyl-Lewis x hexaglycosylceramide (lane 8) was occasionally observed. However, this binding required 2 nmol, while the detection level for NeuAcα3Galβ4(Fucα3)GlcNAcβ3-Galβ4(Fucα3)GlcNAcβ3Galβ4Glcβ1Cer was approximately 1 pmol.

The distinct difference in affinity argue for that the monomeric sialyl-Lewis x ganglioside exhibit features required for bacterial binding, while the dimeric or repetitive form is optimised in its presentation of the terminal sialic acid epitope.

At this stage an open question if the increased binding activity is dependent on the repetitive Lewis x element, or due to the longer carbohydrate chain giving optimal exposure of the binding epitope, as has been described for monoclonal anti-sialyl-Lewis x antibodies (Müthing, J., Spanbroek, R., Peter-Katalinic, J., Hanisch, F.-G., Hanski, C., Hasegawa, A., Unland, F., Lehmann, J., Tschesche, H., and Egge, H. (1996). Isolation and structural characterisation of fucosylated gangliosides with linear poly-N-acetyllactosamineyl chains from human granulocytes. Glycobiology 6, 147-156).

### 6. The receptor specificity of H. pylori for sialylated receptors in ELISA

Since the *H. pylori* wild-type bacteria demonstrate binding to soluble sialyl-Lewis x antigen (Fig. 4), in contrast to the binding patterns as analyzed by the HPTLC binding assays (Fig. 3), there might be an intricate situation where the presence of the BabA-adhesin, such as in the wild type strain, could sterically interfere with binding to the sialyl-Lewis x antigen in solid phase, i.e. to the cell surfaces *in vivo*.

An ELISA assay was constructed where bacterial binding was analysed to immobilised sialylated or sulphated Lewis x antigens. For these experiments, the Lewis b antigen was used as the control. Interestingly, the *babA1A2* mutant binds much more efficiently to the immobilised sialylated receptor compared to the wild type strain, i.e. similar to the results gained from the HPTLC binding analyses. In contrast, binding by the wild type strain to the Lewis b antigen is most efficient, and subsequently completely lost in the *babA1A2* mutant. The drastic difference in binding results for soluble versus immobilized sialylated structures, is suggestive of a situation where interaction with sialylated mucin molecules in the gastric mucus layer (lining the epithelial cells), might be compatible with the binding behaviour of the CCUG 17875 wild type, while tight interaction with cell surface immobilized or presented receptors might be confined to the *babA1A2* mutant. This would subsequently be a situation most similar to the binding pattern of strain CCUG 17874, where lack of the BabA-adhesin, by down regulation or phase shifting, in order to adapt to variations in the local glycosylation patterns, and in efficient binding to sialylated receptors (Fig 3). In these analyses, a weak interaction with the sialyl-Lewis a antigen is also detected by strain CCUG 17875. However, the difference in binding is close to 100-fold lower compared to sialyl-Lewis x antigen. In addition, sulphated derivatives of the Lewis x/a antigens has been demonstrated as efficient selectin-receptors (C.T. Yuen *et al., J. Biol. Chem.,* 269, *1598,* (1994); Brandley *et al., Glycobiology*, 3, 663 (1993). 3'HSO3Lewis a- and 3'HSO3Lewis x - antigen conjugates were analyzed for bacterial binding in ELISA. However, no binding above background was detected suggesting a strict requirement for terminal alfa2.3 linked sialic acid in the receptor epitope, in contrast to the receptor /carbohydrate binding domains of the selectin proteins (Fig. 6).

### 7. Adherence of the H. pylori babA1/babA2-mutant is inhibited by the sialyl-Lewis x antigen

In order to demonstrate that the receptor activity for the *babA1*/*babA2*-mutant observed in the *in situ* adherence experiments by human gastric mucosa (Fig. 1 B, C) and, in addition, by the transgenic "Lewis b" mouse gastric mucosa (Fig. 2 D, E), is dependent on the sialyl-Lewis x antigen (Fig. 3, 4, 5, 6), the present inventors performed inhibition of adherence experiment *in situ*. Similar to the previous inhibition experiments with soluble Lewis B neoglyco-conjugates (Fig. 1, D, E), the inventors pre-incubated the *H. pylori* wild type strain (CCUG 17875) and the *babA1*/*babA2*-mutant bacteria, with the sialyl-Lewis x conjugate, before analyses of bacterial binding *in situ* to both human gastric mucosa and to the "Lewis b" mouse gastric mucosa. Most interestingly, the inhibition experiments with sialyl-Lewis x almost completely prevented binding by babA-mutant bacteria (95% reduction), in both human (Fig. 1F and 7A) and "Lewis b" mouse (Fig. 2F and 7C) gastric mucosa. In contrast, the sialyl-Lewis x inhibition experiments with the *H. pylori* wild-type bacteria to human gastric mucosa results in no reduction in binding (Fig. 1 G and 7A).

However, a slight reduction in binding of approx. 35%, was observed with the *H. pylori* wild-type bacteria to the "Lewis b" mouse gastric mucosa (Fig. 2G and 7C). These experiments suggest that the mutant has functionally induced the binding properties of an adhesin protein that is specific for sialyl-Lewis x antigens, i.e. yet another human specific antigen. The slight difference in binding pattern in between the human and transgenic tissues, suggest that the proportions of expressed Lewis b and sialyl-Lewis x antigens are rather different compared to human gastric mucosa. The humanised glycosylation pattern of the "Lewis b" mouse is driven by a truncated rat intestinal fatty acid binding gene promotor (Fabpi) (P.G. Falk et al, (1995) *PNAS*, **92**, 1515). In contrast, the glycosylation pattern of the human gastric mucosa/host is shaped and modulated by factors generated by the chronic inflammation process, such as cytokines and cell differentiation modulating signal molecules and, in addition, differences due to inflammation stimuli, such as cytokines, are most likely to be expected.

### 8. Receptor epitopes for the babA1/babA2-mutant bacteria are recognised by a monoclonal antibody to the sialyl-Lewis x antigen.

The inhibition results with soluble receptor conjugates demonstrate that most of the binding by the *babA1*/*babA2*-mutant is mediated by the sialyl-Lewis x antigen. However, the results from the HPTLC-binding analyses (Fig. 3) suggest that sialyl-Lewis x derivatives with higher molecular weight, possible of repetitive/dimeric nature are less well recognises by the monoclonal antibody. The present inventors have subsequently analyzed the effect on inhibition of bacterial adherence *in situ*, to human gastric mucosa, by monoclonal antibodies to the sialyl-Lewis x, antigen and to the Lewis b antigen (**Fig. 8**). Pre-incubation of the tissue by the sialyl-Lewis x Mab results in a 40% reduction in binding by the*babA1*/*babA2-mutant* compared to no significant reduction in binding by the wild type strain (Fig. 8). In comparison, the Lewis b antigen MAb results in 78% reduction in binding by the wild type strain, while adherence of the *babA1*/*babA2*-mutant strain was unaffected. The 40 % reduction in binding is convincingly efficient, yet suggest that the monoclonal sialyl-Lewis x antibody demonstrate a more restricted binding specificity compared to the*babA1*/*babA2*-mutant. These results are in analogy with the HPTLC-binding results (Fig. 3), where the dimeric sialyl-Lewis x glycolipid/antigen exhibit higher receptor affinity compared to the monomeric shorter carbohydrate structure.

### 9. Expression of the sialyl-Lewis x antigen and receptors for the babA1/babA2-mutant in gastric mucosa

The sialyl-Lewis x antigen seems to be a binding specificity utilised by normal clinical isolates and most likely highly complementary to the previously described Lewis b antigen receptor. Both receptors arealfa-1.3/4-fucosylated and defined as human (primate) specific antigens. The unique alfa-1.3/4-fucosylation of *H. pylori* receptors, might subsequently explain *H. pylori* as a human (primate) specific pathogen. The difference in terminal substitution, in between these two groups of fucosylated blood group related antigens is extremely interesting, since it relates to distinct differences in the epithelial expression patterns of glycosylation. However, the adult healthy human stomach is considered low in sialylated structures, and the presence of sialyl-Lewis x antigens as *H. pylori* receptors would not appear all that obvious. Nevertheless, increased sialylation levels are most often associated with conditions of inflammation and/or malignant transformation, i.e. cellular states associated with the chronic *H. pylori* infectious process.

In order to investigate the possible relation in between gastric inflammation conditions and expression of *H. pylori* sialyl-Lewis x antigen receptors, the present inventors have screened gastric pinch-biopsies (i.e. very small biopsies) from 20 individuals. The present inventors found almost no sialyl-Lewis x dependent binding by the *babA1*/*babA2*-mutant in 10 of the individual pinch-biopsies *in situ,* low and scattered binding in 7 of individuals, and good binding in biopsies of 3 individuals. However, these pinch-biopsies were obtained from routine screening of patients and almost all of these patients were without serious symptoms. One of the three high binding pinch-biopsies was chosen for the experiments in Fig. 1 and Fig. 7A. Six of the pinch-biopsies described above were evaluated for "level of inflammatory cell infiltration". The (four) sections that confer low or modest binding of the *babA1*/*babA2*-mutant were found to be of "low inflammatory cell infiltration", as illustrated by the *in situ* adherence analyses of biopsy no. 9 (Fig. 7B), where binding by the *babA1*/*babA2*-mutant is much reduced. In contrast, the two biopsies that confer good binding by the *babA1*/*babA2*-mutant were of "moderate level of inflammatory cell infiltration". This is in exemplified by biopsy no.12 (Fig 7A). These results suggest a correlation between binding by the *babA1*/*babA2*-mutant and the degree of inflammatory cell infiltration in the gastric tissue/level of inflammation response.

It is of course difficult, based on these results to define when or where the sialyl-Lewis x receptors will emerge, but the present inventors suggest that it might be during the process of chronic atrophic gastritis, when simultaneously the mucus layer gets thinner and it might be beneficial for the bacteria to adhere more tightly to the cells, in order to avoid the acidic environment.

Based on the results, there might be the situation where *H. pylori* is targeted to the surface epithelial lining/gastric pit region, by adherence mediated by the Lewis b antigens. During the chronic infection and inflammation process, the epithelial cells will response by up-regulation of the sialylation levels. This event/process then provides a second receptor level i.e. a consecutive or two-step mechanism for the microbial attachment process, and adhesion to the sialyl-Lewis x antigens, in the gastric pit region. A 2-step dependent mechanism for adhesion could be of great importance for the microbe, if initial targeting to the surface mucus cells is mediated by the Lewis b antigens, followed by an intimate contact with the cellular membrane mediated by sialylated glycolipids.

A stepwise attachment mechanism might be extremely useful for the microbes, and could by triggering the infectious process induce a second level of cellular receptor expression, in order to promote adherence. Such mechanisms would direct them to a slightly shifted ecological niche during the chronic inflammation process, and subsequently driving the inflammation process further to development of acid peptic disease and/or malignant transformation. Another possible explanation might be to avoid initial interaction with sialylated inflammatory cells such as PMN-cells/granulocytes. Lectin mediated interaction with neutrophils has been demonstrated to activate oxidative bursts reactions (D. Danielsson, et al., *Scand-J-Gastroenterol*, 1994, 29, 2, 128-32). The receptor for such mechanisms has not been identified yet, but considering that human granulocytes are often used as model cells for sialyl-Lewis x antigen based selectin-interactions/adherence studies, there is a definite possibility that reactive interactions with granulocytes might be triggered by the interaction with sialyl-Lewis x antigen/receptor.

Much of the present experimental results and understanding has been made possible due to the bindings results generated by the *babA1A2*-"double"mutant (DM), previously defined by the inventors. Since the double-mutant is devoid of all Lewis b antigen binding properties, the specific binding by sialyl-Lewis x antigens in the gastric pit region could be demonstrated. The presence of the BabA adhesin (i.e. the wild-type) seems to somehow sterically interfere and prevent binding by the sialyl-Lewis x antigen *in situ*, as revealed by the present results of binding experiments in solid phase to sialylated receptors on HPLTC-plates and in ELISA.

The present inventors have demonstrated surprising sialyl-Lewis x antigen dependent binding modes, as a consequence of the gastric tissue inflammation response. Such signalling might be key-events for the *H. pylori* chronic infection cycle, supporting the chronic inflammation response and development of acid peptic disease/gastric cancer.

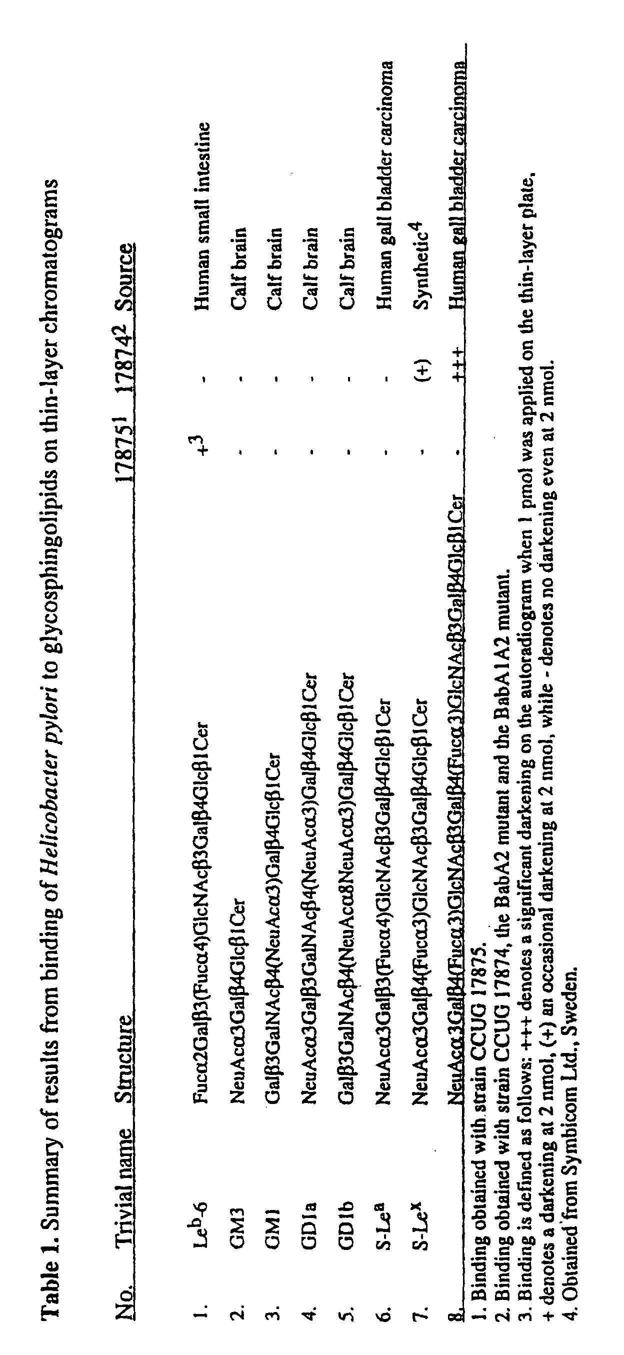

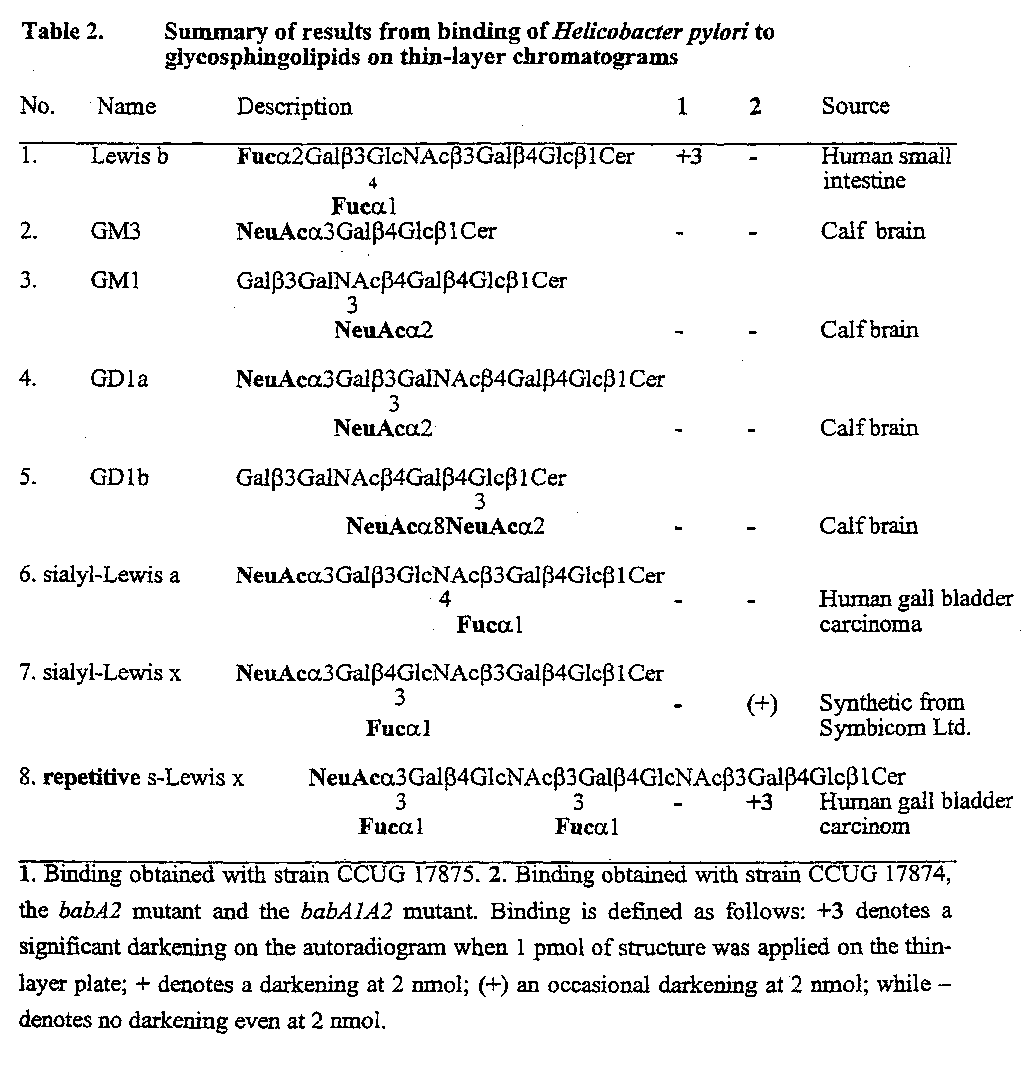

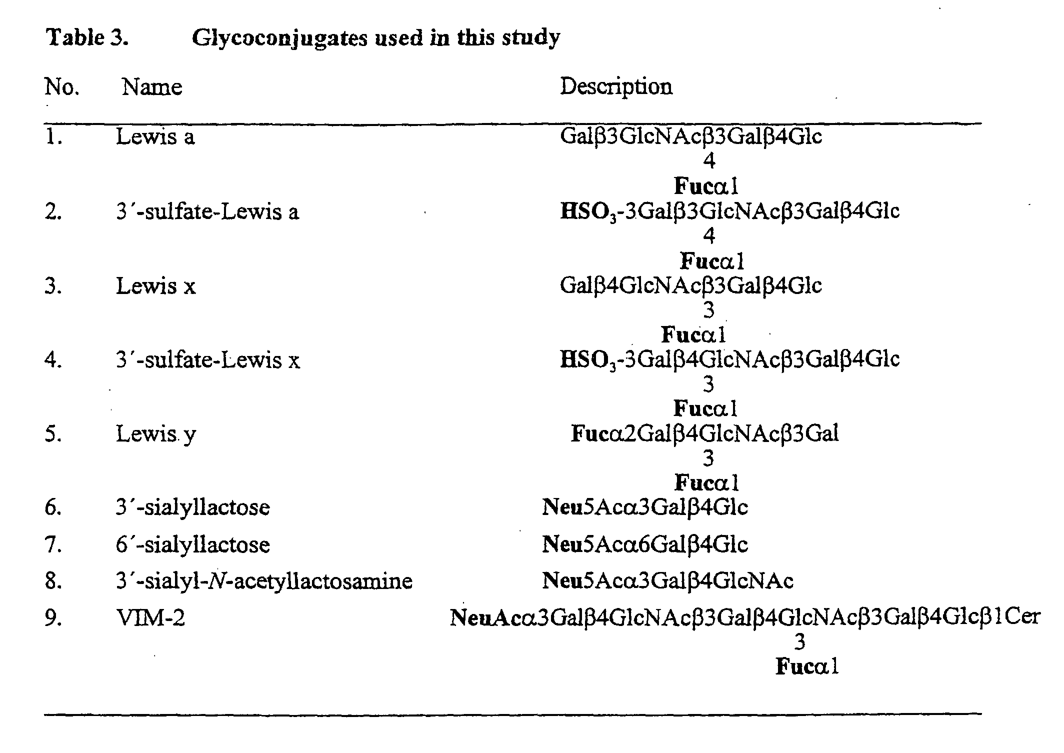

## Claims

1. Use of a fucosylated sialylated N-acetyl lactosamine structure and/or a sialyl-Lewis antigen carbohydrate structure for the preparation of a pharmaceutical composition for the treatment or prophylaxis in humans of infections by *Helicobacter pylori* of the human gastrointestinal mucosa.

2. Use according to claim 1, in which the sialyl-Lewis antigen carbohydrate structure is capable of binding to adhesins present on the surface of *H. pylori*.

3. Use according to claim 1 or 2, in which the sialyl-Lewis antigen carbohydrate structure is capable of inhibiting or substantially reducing the adhesion of *H. pylori* to epithelial cells of a histological section of human gastrointestinal mucosa.

4. Use according to any one of claims 1 - 3, in which the sialyl-Lewis antigen is chosen among sialyl-Lewis x and sialyl-Lewis a.

5. Use according to any one of claims 1 - 3, in which the sialyl-Lewis antigen is chosen among dimeric or repetitive sialyl-Lewis x and dimeric or repetitive sialyl-Lewis a.

6. Use according to any one of claims 1 - 5, in which the gastrointestinal infections by *H. pylori* comprise gastritis, chronic active gastritis, gastric ulcers, duodenal ulcers, gastric adenocarcinoma, and gastric lymphoma.

7. Use according to any of the claims 1-6, in which the sialyl-Lewis antigen carbohydrate structure is bound to an inert substrate, preferably for long term release in the gastrointestinal tract.

## Patentansprüche

1. Verwendung einer fucosylierten sialylierten N-Acetyl-lactosamin-Struktur und/oder einer Sialyl-Lewis-Antigen-Kohlehydratstruktur zur Herstellung einer pharmazeutischen Zusammensetzung zur Behandlung oder Prophylaxe bei Menschen von Infektionen der menschlichen Magen-Darm-Schleimheit mit Helicobacter pylori.

2. Verwendung nach Anspruch 1, bei der die Sialyl-Lewis-Antigen-Kohlehydratstruktur in der Lage ist, an Adhäsine, die an der Oberfläche von H. pylory vorhanden sind, zu binden.

3. Verwendung nach Anspruch 1 oder 2, bei der die Sialyl-Lewis-Antigen-Kohlehydratstruktur in der Lage ist, die Haftung von H. pylory an Epithelzellen eines histologischen Bereichs der menschlichen Magen-Darm-Schleimhaut zu hemmen oder wesentlich zu verringern.

4. Verwendung nach einem der Ansprüche 1 bis 3, bei der das Sialyl-Lewis-Antigen unter Sialyl-Lewis x und Sialyl-Lewis a ausgewählt wird.

5. Verwendung nach einem der Ansprüche 1 bis 3, bei der das Sialyl-Lewis-Antigen unter dimerem oder repetitivem Sialyl-Lewis x und dimerem oder repetitivem Sialyl-Lewis a ausgewählt wird.

6. Verwendung nach einem der Ansprüche 1 bis 5, bei der die gastrointestinalen Infektionen mit H. pylori Gastritis, chronische aktive Gastritis, Magengeschwüre, Zwölffingerdarm-Geschwüre, Magen-Adenokarzinome und Magen-Lymphome aufweisen.

7. Verwendung nach einem der Ansprüche 1 bis 6, bei der die Sialyl-Lewis Antigen-Kohlehydratstruktur an ein inertes Substrat, bevorzugt zur Langzeit-Freisetzung im Magen-Darm-Trakt, gebunden wird.

## Revendications

1. Application d'une structure de N-acétyllactosamine sialylée et fucosylée et/ou d'une structure d'hydrate de carbone antigène de Lewis-sialyle à la préparation d'une composition pharmaceutique pour le traitement où la prophylaxie humaine des infections par *Helicobacter pylori* de la muqueuse gastro-intestinale humaine.

2. Application selon la revendication 1, dans laquelle la structure d'hydrate de carbone antigène de Lewis-sialyle peut présenter une liaison à des adhésines présentes à la surface de *H. pylori*.

3. Application selon la revendication 1 ou 2, dans laquelle la structure d'hydrate de carbone antigène de Lewis-sialyle permet l'inhibition ou la réduction notable de l'adhérence de *H. pylori* aux cellules épithéliales d'une section histologique de la muqueuse gastro-intestinale humaine.

4. Application selon l'une quelconque des revendications 1 à 3, dans laquelle l'antigène de Lewis-sialyle est choisi parmi les antigènes de Lewis-sialyle x et de Lewis-sialyle a.

5. Application selon l'une quelconque des revendications 1 à 3, dans laquelle l'antigène de Lewis-sialyle est choisi parmi l'antigène de Lewis-sialyle x dimère ou répétitif et l'antigène de Lewis-sialyle dimère ou répétitif.

6. Application selon l'une quelconque des revendications 1 à 5, dans laquelle les infections gastro-intestinales par *H. pylori* comprennent la gastrite, la gastrite active chronique, les ulcères gastriques, les ulcères du duodénum, l'adénocarcinome gastrique et le lymphome gastrique.

7. Application selon l'une quelconque des revendications 1 à 6, dans laquelle la structure d'hydrate de carbone d'antigène de Lewis-sialyle est liée à un substrat inerte, de préférence permettant une libération sur une longue durée dans le système gastro-intestinal.
